(19) Europäisches Patentamt
European Patent Office
Office européen des brevets

(11) **EP 3 801 667 B1**

(12) **EUROPÄISCHE PATENTSCHRIFT**

(45) Veröffentlichungstag und Bekanntmachung des Hinweises auf die Patenterteilung:
**20.11.2024  Patentblatt 2024/47**

(21) Anmeldenummer: **19729727.8**

(22) Anmeldetag: **06.06.2019**

(51) Internationale Patentklassifikation (IPC):
*A61M 60/546* (2021.01)   *A61M 60/178* (2021.01)
*A61M 60/216* (2021.01)   *A61M 60/411* (2021.01)

(52) Gemeinsame Patentklassifikation (CPC):
**A61M 60/546; A61M 60/178; A61M 60/216; A61M 60/411**

(86) Internationale Anmeldenummer:
**PCT/EP2019/064802**

(87) Internationale Veröffentlichungsnummer:
**WO 2019/234162 (12.12.2019 Gazette 2019/50)**

(54) **VERFAHREN ZUR BESTIMMUNG EINES FLUID-GESAMTVOLUMENSTROMS IM BEREICH EINES IMPLANTIERTEN, VASKULÄREN UNTERSTÜTZUNGSSYSTEMS SOWIE IMPLANTIERBARES VASKULÄRES UNTERSTÜTZUNGSSYSTEM**

METHOD FOR DETERMINING A FLUID TOTAL VOLUME FLOW IN THE REGION OF AN IMPLANTED VASCULAR SUPPORT SYSTEM AND IMPLANTABLE VASCULAR SUPPORT SYSTEM

PROCÉDÉ POUR DÉTERMINER UN DÉBIT VOLUMIQUE TOTAL DE FLUIDE DANS LA ZONE D'UN SYSTÈME DE SUPPORT VASCULAIRE IMPLANTÉ AINSI QU'UN SYSTÈME DE SUPPORT VASCULAIRE IMPLANTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018   DE 102018208879**

(43) Veröffentlichungstag der Anmeldung:
**14.04.2021   Patentblatt 2021/15**

(73) Patentinhaber: **Kardion GmbH**
**70376 Stuttgart (DE)**

(72) Erfinder:
• **BAUMBACH, Hardy**
**70376 Stuttgart (DE)**

• **SCHNEIDER, Karin**
**71083 Herrenberg (DE)**
• **SCHELLENBERG, Inga**
**70191 Stuttgart (DE)**
• **BUDDE, Martina**
**76229 Karlsruhe (DE)**
• **SCHLEBUSCH, Thomas, Alexander**
**71272 Renningen (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
**WO-A1-98/43688     WO-A2-2012/112378**

**Beschreibung**

Die Erfindung betrifft ein Verfahren zur Bestimmung eines Fluid-Gesamtvolumenstroms im Bereich eines implantierten, vaskulären Unterstützungssystems, eine Verarbeitungseinheit sowie ein implantierbares, vaskuläres Unterstützungssystem. Die Erfindung findet insbesondere Anwendung bei (voll-)implantierten Linksherz-Unterstützungssystemen (LVAD).

Ein derartiges Verfahren, eine derartige Verarbeitungseinheit und ein derartiges implantierbares, vaskuläres Unterstützungssystem sind aus der WO 2012/112378 A2 und aus der WO 98/43688 A1 bekannt.

Implantierte Linksherz-Unterstützungssysteme (LVAD) existieren hauptsächlich in zwei Ausführungsvarianten. Einerseits gibt es (perkutane) minimalinvasive Linksherz-Unterstützungssysteme. Die zweite Variante sind unter der Brustkorböffnung invasiv implantierte Linksherz-Unterstützungssysteme. Die Variante nach erste Variante fördert Blut direkt aus dem linken Ventrikel in die Aorta, da das (perkutane) minimalinvasive Linksherz-Unterstützungssystem mittig in der Aortenklappe positioniert ist. Die zweite Variante fördert das Blut aus dem linken Ventrikel über einen Bypass-Schlauch in die Aorta.

Die Aufgabe eines kardialen Unterstützungssystems ist die Förderung von Blut. Hierbei hat das sog. Herz-Zeit-Volumen (HZV, üblicherweise angegeben in Liter pro Minute) eine hohe klinische Relevanz. Das Herz-Zeit-Volumen betrifft hierbei mit anderen Worten den Gesamtvolumenstrom an Blut (aus einem Ventrikel), insbesondere vom linken Ventrikel hin zur Aorta. Entsprechend eingängig ist das Bestreben, diesen Parameter als Messwert während des Betriebs eines kardialen Unterstützungssystems zu erheben.

Je nach Unterstützungsgrad, der den Anteil des durch ein Fördermittel wie etwa eine Pumpe des Unterstützungssystems geförderten Volumenstroms zum Gesamtvolumenstrom an Blut vom Ventrikel hin zur Aorta beschreibt, gelangt ein gewisser Volumenstrom über den physiologischen Weg durch die Aortenklappe in die Aorta. Das Herz-zeit-Volumen bzw. der Gesamtvolumenstrom ($Q_{HZV}$) vom Ventrikel hin zur Aorta ist demnach üblicherweise die Summe aus Pumpenvolumenstrom ($Q_p$) und Aortenklappen-Volumenstrom ($Q_a$).

Ein etabliertes Verfahren zur Bestimmung des Herz-Zeit-Volumens ($Q_{HZV}$) im klinischen Umfeld ist die Verwendung von Dilutionsverfahren, die jedoch alle auf einen transkutan eingeführten Katheter setzen und daher nur während einer Herzoperation Herz-Zeit-Volumen-Messdaten liefern können. Ein etabliertes Verfahren zur Messung des Pumpenvolumenstroms ($Q_p$) ist die Korrelation aus den Betriebsparametern des Unterstützungssystems, vor allem der elektrischen Leistungsaufnahme, eventuell ergänzt um weitere physiologische Parameter wie den Blutdruck. Auch die Integration dedizierter Ultraschall-Messtechnik in ein Unterstützungssystem wurde bereits vorgeschlagen.

Eine (voll-)implantierte Erfassung des Herz-Zeit-Volumens, also von $Q_{HZV}$, insbesondere durch das Unterstützungssystem selbst, war bisher noch nicht vorgeschlagen oder realisiert worden. Voll-implantiert bedeutet hierbei insbesondere, dass die für die Erfassung erforderlichen Mittel sich vollständig im Körper des Patienten befinden und dort verbleiben. Dies ermöglicht es, das Herz-Zeit-Volumen auch außerhalb einer Herzoperation zu erfassen.

Aufgabe der Erfindung ist es, ein verbessertes Verfahren zur Bestimmung eines Fluid-Gesamtvolumenstroms im Bereich eines implantierten, vaskulären Unterstützungssystems anzugeben und ein verbessertes implantierbares, vaskuläres Unterstützungssystem zu schaffen.

Insbesondere ist es eine Aufgabe der Erfindung, ein Verfahren zur Bestimmung eines Fluid-Gesamtvolumenstroms im Bereich eines implantierten, vaskulären Unterstützungssystems anzugeben und ein implantierbares, vaskuläres Unterstützungssystem zu schaffen, mittels dessen ein Fluid-Gesamtvolumenstrom in einem mit Blut durchströmten Bereich in einem menschlichen oder tierischen Körper bestimmt werden kann, in dem das vaskuläre Unterstützungssystem implantiert bzw. angeordnet ist.

Diese Aufgabe wird durch das in Anspruch 1 angegebene Verfahren, die in Anspruch 7 angegebene Verarbeitungseinheit und das in Anspruch 8 angegebene implantierbare vaskuläre Unterstützungssystem gelöst.

Vorteilhafte Ausführungsformen der Erfindung sind insbesondere in den abhängigen Ansprüchen angegeben.

Das in Anspruch 1 angegebene Verfahren zur Bestimmung eines Fluid-Gesamtvolumenstroms im Bereich eines implantierten, vaskulären Unterstützungssystems, umfasst folgende Schritte:

a) Bestimmen einer Referenztemperatur des Fluids,
b) Bestimmen einer Motortemperatur eines Elektromotors des Unterstützungssystems,
c) Bestimmen der thermischen Verlustleistung des Elektromotors,
d) Ermitteln des Fluid-Gesamtvolumenstroms unter Verwendung der Referenztemperatur, der Motortemperatur und der thermischen Verlustleistung des Elektromotors.

Das vaskuläre Unterstützungssystem ist bevorzugt ein kardiales Unterstützungssystem, besonders bevorzugt ein ventrikuläres Unterstützungssystem. Mit dem Gesamtvolumenstrom ist insbesondere der gesamte Volumenstrom durch ein Blutgefäß bzw. durch einen Querschnitt des Blutgefäßes gemeint.

Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem, bevorzugt um die Aorta. Vorzugsweise dient das Verfahren zur Bestimmung eines Fluid-Gesamtvolumenstroms aus einem Ventrikel eines Herzens, insbesondere von einem (linken) Ventrikel eines Herzens hin zur Aorta im Bereich eines (voll-)implantierten, (links-)ventrikulären (Herz-)Unterstützungssystems. Bei dem Fluid handelt es sich regelmäßig um Blut. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet.

Das Verfahren ist insbesondere zur Bestimmung des gesamten Herz-Zeit-Volumens (HZV, Formelzeichen $Q_{HZV}$) eines Patienten, insbesondere mit (voll-)implantiertem linksventrikulären Herzunterstützungssystem (LVAD) in Aortenklappenposition und/oder durch das Unterstützungssystem selbst geeignet. Das Verfahren basiert insbesondere auf (thermisch) anemometrischen (Mess-)Prinzipien zur Flussmessung. Grundprinzip ist dabei, dass ein strömendes Medium einen heißen Körper in Abhängigkeit der Strömungsgeschwindigkeit kühlt. Das Verfahren ermöglich in vorteilhafter Weise, dass das Herz-Zeit-Volumen auch außerhalb des OP-Szenarios mit vergleichbarer Qualität wie bei Verwendung eines Dilutionskatheters zur Verfügung gestellt werden kann. Dies ist von besonderem Vorteil, da das Herz-Zeit-Volumen ($Q_{HZV}$) eine größere klinische Relevanz als der meist verwendete Pumpenvolumenstrom ($Q_p$) hat, der nur den Fluss durch das Unterstützungssystem selbst quantifiziert.

Ein besonderer Vorteil des Verfahrens besteht darin, dass anders als bei anemometrischen Verfahren üblich kein gesondertes Heizelement zur Erzeugung des zu messenden Wärmeflusses benötigt wird. Vielmehr kann die ohnehin am Elektromotor des LVAD auftretende thermische Verlustleistung zur anemometrischen Flussmessung genutzt werden. Bevorzugt wird zur Bestimmung des Fluid-Gesamtvolumenstroms kein (gesondertes) Heizelement (außer dem Elektromotor) verwendet. Mit anderen Worten ausgedrückt der Elektromotor das einzige Heizelement das bei der hier vorgeschlagenen Lösung zum Einsatz kommt. Insbesondere wird bei der hier vorgeschlagenen Lösung die am und/oder im Elektromotor des Unterstützungssystems auftretende thermische Verlustleistung zur (thermisch) anemometrischen bzw. kalorimetrischen Flussmessung verwendet. Weiterhin bevorzugt ist es, dass das Unterstützungssystem kein (gesondertes) Heizelement (außer dem Elektromotor) aufweist.

In Schritt a) wird eine Referenztemperatur des Fluids bestimmt, insbesondere gemessen. Bevorzugt wird die Referenztemperatur durch einen Referenztemperatursensor bestimmt, der besonders bevorzugt Bestandteil des Unterstützungssystems ist. Der Referenztemperatursensor kann beispielsweise in und/oder an einer (Einlauf-)Kanüle des Unterstützungssystems angeordnet sein. Die Referenztemperatur stellt üblicherweise eine Hintergrundtemperatur des Fluids dar, mit anderen Worten eine Fluidtemperatur, die insbesondere nicht durch die thermische Verlustleistung des Elektromotors beeinflusst ist.

In Schritt b) erfolgt ein Bestimmen, insbesondere Messen einer Motortemperatur eines Elektromotors des Unterstützungssystems. Der Elektromotor kann Bestandteil einer Strömungsmaschine bzw. einer Pumpe des Unterstützungssystems sein. Vorzugsweise ist das Unterstützungssystem so an oder in der Fluidströmung angeordnet, dass ein Wärmestrom von dem Unterstützungssystem, insbesondere von dessen Elektromotor, an die Fluidströmung abgegeben werden kann. Unter der Motortemperatur kann auch eine Innentemperatur oder (Außen-)Oberflächentemperatur des Unterstützungssystems, insbesondere im Bereich des Elektromotors, verstanden werden, die insbesondere einen bevorzugt unmittelbaren Rückschluss auf die Temperatur des Elektromotors, insbesondere auf die Temperatur eines Wicklungspakets des Elektromotors zulässt.

Das Unterstützungssystem ist bevorzugt so implantiert, dass es sich zumindest teilweise, bevorzugt vollständig oder mit mindestens 50 %, besonders bevorzugt mindestens 85 % oder sogar mindestens 95 % seiner (Außen-)Oberfläche in der Fluidströmung befindet. Weiterhin bevorzugt befindet sich das Unterstützungssystem entlang mindestens 50 %, besonders bevorzugt mindestens 85 % oder sogar mindestens 95 % seiner Länge in der Fluidströmung. Bevorzugt befindet sich ein Ende des Unterstützungssystems, im Bereich dessen bzw. an dem sich der Elektromotor befindet, zumindest teilweise in der Aorta. Weiterhin bevorzugt befindet sich das gegenüberliegende Ende des Unterstützungssystems, im Bereich dessen bzw. an dem sich eine (Einlauf-)Kanüle des Unterstützungssystems befindet, zumindest teilweise in einem (dem linken) Ventrikel des Herzens. Weiterhin bevorzugt ist das Unterstützungssystem zumindest teilweise, bevorzugt vollständig oder mit mindestens 50 %, besonders bevorzugt mindestens 85 % oder sogar mindestens 95 % seiner (Außen-)Oberfläche in einem Blutgefäß, wie etwa einer Arterie, insbesondere der Aorta angeordnet. Besonders bevorzugt ist das Unterstützungssystem so implantiert, dass es sich (vollständig) in der (deszendenten) Aorta befindet.

In Schritt c) wird die thermische Verlustleistung des Elektromotors bestimmt. Bevorzugt wird die thermische Verlustleistung des Elektromotors mit einem Stromsensor bestimmt, der vorzugsweise einen elektrischen Strom des Elektromotors misst.

In Schritt d) wird der Fluid-Gesamtvolumenstrom unter Verwendung der Referenztemperatur, der Motortemperatur und der thermischen Verlustleistung des Elektromotors bestimmt. Bevorzugt wird in Schritt d) unter Zuhilfenahme von mindestens einer Wärmeübertragungsvorschrift, mindestens einem Wärmeübertragungskoeffizienten, mindestens einem Kalibrierfaktor und/oder mindestens einem Blutgefäßquerschnitt, insbesondere Aortenquerschnitt der Fluid-Gesamtvolumenstrom in Abhängigkeit von der Referenztemperatur, der Motortemperatur und der thermischen Verlustleistung des Elektromotors bestimmt.

Nach einer vorteilhaften Ausgestaltung wird vorgeschlagen, dass die Referenztemperatur, insbesondere räumlich und/oder zeitlich vor einer Erwärmung des Fluids durch den Elektromotor gemessen wird. Bevorzugt ist ein Referenztemperatursensor beabstandet zu dem Elektromotor, insbesondere stromauf des Elektromotor, vorzugsweise an einer (Einlauf-)Kanüle des Unterstützungssystems angeordnet. Besonders bevorzugt ist der Referenztemperatursensor im Bereich eines und/oder an einem dem Elektromotor gegenüberliegenden Ende(s) der (Einlauf-)Kanüle angeordnet.

Nach einer vorteilhaften Ausgestaltung wird vorgeschlagen, dass die Motortemperatur des Elektromotors an einer Oberfläche gemessen wird, an der das Fluid entlangströmt. Bei der Oberfläche handelt es sich in der Regel um eine (Außen-)Oberfläche des Unterstützungssystems, die in Kontakt mit dem Fluid steht. Die Motortemperatur kann beispielsweise mit einem Motortemperatursensor gemessen werden, der an einer (Außen-)Oberfläche des Unterstützungssystems im Bereich des (innenliegenden) Elektromotors angeordnet ist. Alternativ hierzu kann die Motortemperatur des Elektromotors im Motorinneren gemessen werden. Hierzu kann ein Motortemperatursensor im Inneren des Elektromotors angeordnet sein.

Nach einer vorteilhaften Ausgestaltung wird vorgeschlagen, dass in Schritt d) in Abhängigkeit von Kalibrierdaten, der Referenztemperatur, der Motortemperatur und der thermischen Verlustleistung des Elektromotors eine Strömungsgeschwindigkeit des Fluids bestimmt, insbesondere berechnet wird. Die Kalibrierdaten umfassen bevorzugt eine charakteristische Länge (z. B. Rohrdurchmesser, ggf. angenähert im Bereich der Aortenklappe), eine kinematischen Viskosität des Fluids, eine Temperaturleitfähigkeit des Fluids, eine Wärmeleitfähigkeit des Fluids und/oder eine mit Fluid benetzte (Ober-)Fläche des Unterstützungssystems.

Nach einer vorteilhaften Ausgestaltung wird vorgeschlagen, dass in Schritt d) weiterhin eine ermittelte Querschnittsgeometrie einer Aorta im Bereich des implantierten, vaskulären Unterstützungssystems berücksichtigt wird. Vorzugsweise wird ein (durchströmbarer) Querschnitt der Aorta im Bereich des Unterstützungssystems berücksichtigt. Dieser Wert kann vom Arzt beispielsweise durch Ultraschall oder Computertomografie ermittelt werden. Der Fluid-Gesamtvolumenstrom bzw. das Herz-Zeit-Volumen kann besonders vorteilhaft in Abhängigkeit der Strömungsgeschwindigkeit des Fluids, des (durchströmbaren) Querschnitts der Aorta und einem (geschwindigkeitsabhängigen) Kalibrierfaktor bestimmt, insbesondere berechnet werden. Der (geschwindigkeitsabhängige) Kalibrierfaktor kann z. B. durch eine Kalibrierung im Rahmen der Implantation ermittelt werden, beispielsweise durch Einsatz eines Dilutionskatheters als Referenzstandard.

Nach einer vorteilhaften Ausgestaltung wird vorgeschlagen, dass weiterhin ein Fluid-Volumenstrom bestimmt wird, der durch das Unterstützungssystems strömt. Dies betrifft mit anderen Worten insbesondere einen Fluid-Volumenstrom der nur durch das Unterstützungssystems selbst hindurch fließt. Bei diesem Fluid-Volumenstrom handelt es sich üblicherweise um den sog. Pumpenvolumentstrom ($Q_p$), der nur den Fluss durch das Unterstützungssystem selbst quantifiziert. Ist dieser Wert zusätzlich zu dem Gesamtvolumenstrom bzw. Herz-Zeit-Volumen ($Q_{HZV}$) bekannt, so kann aus dem Verhältnis von $Q_p$ zu $Q_{HZV}$ (d.h. $Q_p/Q_{HZV}$) der sogenannte Unterstützungsgrad berechnet werden. Zur Bestimmung des Pumpenvolumenstroms kann ein Eingangs im Zusammenhang mit dem Stand der Technik erörtertes, etabliertes Verfahren zur Messung des Pumpenvolumenstroms verwendet werden.

Bevorzugt wird der in Schritt d) ermittelte Fluid-Gesamtvolumenstrom beispielsweise in einem Schritt e) als Regelparameter für das Unterstützungssystem bereitgestellt. Eine Verarbeitungseinheit des Unterstützungssystems kann diesen Regelparameter als Ausgangsgröße, insbesondere einer Steuereinheit des Unterstützungssystems, dass vorzugsweise die Leistung des Elektromotors und damit insbesondere auch die (Blut-)Förderleistung des Unterstützungssystems regelt, bereitstellen.

Nach einem weiteren Aspekt wird eine Verarbeitungseinheit vorgeschlagen, eingerichtet zur Durchführung eines hier vorgeschlagenen Verfahrens und aufweisend einen Speicher, in dem Kalibrierdaten hinterlegt sind. Alternativ oder zusätzlich zu den Kalibrierdaten kann in dem Speicher auch mindestens ein (geschwindigkeitsabhängiger) Kalibierfaktor und/oder ein thermisches Modell des Elektromotors hinterlegt sein. Darüber hinaus kann die Verarbeitungseinheit einen Mikroprozessor umfassen, der auf den Speicher zugreifen kann. Die Verarbeitungseinheit empfängt vorzugsweise Daten von einem Referenztemperatursensor, einem Motortemperatursensor und/oder einem Stromsensor.

Nach einem weiteren Aspekt wird ein implantierbares, vaskuläres Unterstützungssystem vorgeschlagen, umfassend:

- einen Referenztemperatursensor zur Bestimmung einer Referenztemperatur eines Fluids,
- einen Elektromotor,
- einen Motortemperatursensor zur Bestimmung einer Motortemperatur des Elektromotors,
- einen Stromsensor zur Bestimmung zumindest des Stromflusses durch den Elektromotor oder der thermischen Verlustleistung des Elektromotors.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere der Referenztemperatursensor, der Motortemperatursensor und der Stromsensor sich vollständig im Körper des Patienten befinden und dort verbleiben. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Der Stromsensor dient zur Bestimmung des Stromflusses durch den Elektromotor und/oder der thermischen Verlustleistung des Elektromotors. Bevorzugt misst der Stromsensor den Stromfluss durch den Elektromotor und berechnet hieraus die Verlustleistung des Elektromotos. Wenn der Stromsensor lediglich den Stromfluss als Ausgangsgröße liefert, ist insbesondere vorgesehen, dass der Stromfluss in einer Verarbeitungseinheit des Unterstützungssystems in die Verlustleistung des Elektromotors umgerechnet wird.

Weiterhin bevorzugt umfasst das Unterstützungssystem eine Kanüle, insbesondere Einlaufkanüle und eine Strömungsmaschine, wie etwa eine Pumpe. Der Elektromotor ist dabei regelmäßig ein Bestandteil der Strömungsmaschine. Der Elektromotor treibt dann die Strömungsmaschine für das Fördern des Fluids an. Die (Einlauf-)Kanüle ist vorzugsweise so eingerichtet, dass sie im implantierten Zustand Fluid aus einem (linken) Ventrikel eines Herzens hin zu der Strömungsmaschine führen kann. Durch die Kanüle kann das Fluid zu der Strömungsmaschine geführt werden. Die die Kanüle ist dabei bevorzugt für das Führen von Fluid in Form von Blut aus einem (linken) Ventrikel eines Herzens in eine Aorta ausgelegt.

Das Unterstützungssystem ist vorzugsweise länglich und/oder schlauchartig gebildet. Bevorzugt sind die Einlaufkanüle und die Strömungsmaschine im Bereich einander gegenüberliegender Enden des Unterstützungssystems angeordnet.

Der Referenztemperatursensor kann an oder in der Nähe eines von der Strömungsmaschine beabstandeten Bereichs der Kanüle angeordnet sein. Insbesondere kann der Referenztemperatursensor an oder in der Nähe eines dem Elektromotor abgewandten Bereichs der Kanüle angeordnet sein. Besonders bevorzugt ist der Referenztemperatursensor ein einem distalen Ende der Kanüle angeordnet, d.h. dort wo das Blut aus einem Ventrikel in die Kanüle zuströmt.

Das Unterstützungssystem kann eine schlauchartige längliche Struktur mit einem Kanülenabschnitt aufweisen, in dem die Kanüle ausgebildet ist, und mit einem an den Kanülenabschnitt angeschlossenen Motorgehäuseabschnitt, in dem der Elektromotor in einem Motorgehäuse angeordnet ist.

Von Vorteil ist es, wenn der Referenztemperatursensor in einem von dem Motorgehäuseabschnitt beabstandeten Bereich des Kanülenabschnitts angeordnet ist. Der Elektromotor ist bevorzugt in einem in der Aorta mit Blut umströmbaren Motorgehäuse angeordnet.

Das Unterstützungssystem kann weiterhin eine Verarbeitungseinheit umfassen, die eingerichtet ist zur Bestimmung eines Fluid-Gesamtvolumenstroms im Bereich des Unterstützungssystems unter Verwendung der Referenztemperatur, der Motortemperatur und der thermischen Verlustleistung des Elektromotors. Bevorzugt ist das Unterstützungssystem zur Durchführung eines hier vorgeschlagenen Verfahrens eingerichtet.

Die im Zusammenhang mit dem Verfahren erörterten Details, Merkmale und vorteilhaften Ausgestaltungen können entsprechend auch bei der hier vorgestellten Verarbeitungseinheit und/oder dem Unterstützungssystem auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Es zeigen schematisch:

Fig. 1a    ein perkutanes, minimalinvasives Linksherz-Unterstützungssystem,

Fig. 1b    ein unter der Brustkorböffnung invasiv implantiertes Linksherz-Unterstützungssystem,

Fig. 2    ein implantiertes, vaskuläres Unterstützungssystem,

Fig. 3    eine Anordnung eines implantierten, vaskulären Unterstützungssystems,

Fig. 4    eine Komponentenarchitektur eines Unterstützungssystems,

Fig. 5    eine Veranschaulichung eines Wärmeflusses,

Fig. 6    eine Veranschaulichung eines Temperaturverlaufs, und

Fig. 7      eine weitere Veranschaulichung eines Temperaturverlaufs.

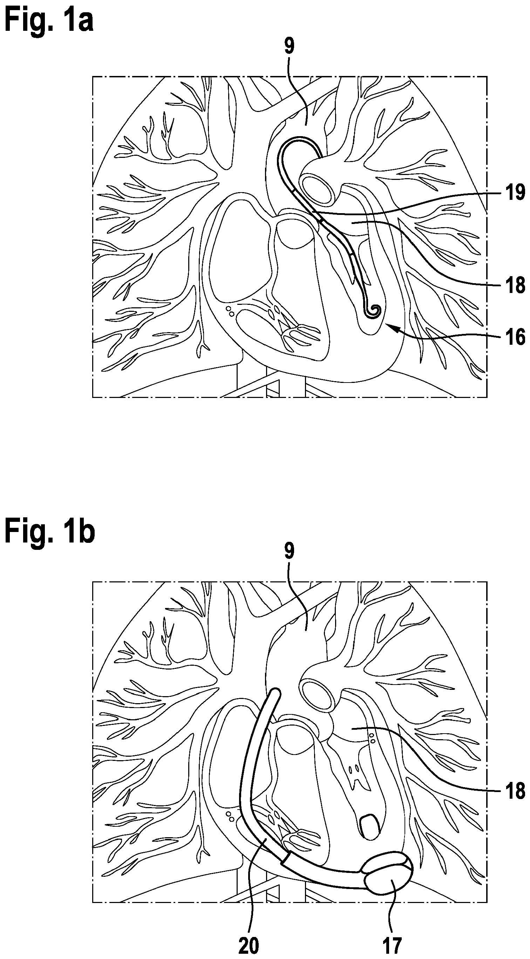

Implantierte Linksherz-Unterstützungssysteme (LVAD) existieren hauptsächlich in zwei Ausführungsvarianten, wie sie in den Fig. 1a und 1b gezeigt sind. Fig. 1a zeigt ein (perkutanes) minimalinvasives Linksherz-Unterstützungssystem 16, während Fig. 1b ein unter der Brustkorböffnung invasiv implantiertes Linksherz-Unterstützungssystem 17 zeigt. Die Variante nach Fig. 1a fördert Blut direkt aus dem linken Ventrikel 18 in die Aorta 9, da das (perkutane) minimalinvasive Linksherz-Unterstützungssystem 16 mittig in der Aortenklappe 19 positioniert ist. Die Variante nach Fig. 1b fördert das Blut aus dem linken Ventrikel 18 über einen Bypass-Schlauch 20 in die Aorta 9.

Je nach Unterstützungsgrad, der den Anteil des durch ein Fördermittel wie etwa eine Pumpe des Unterstützungssystems geförderten Volumenstroms zum Gesamtvolumenstrom an Blut vom Ventrikel 18 hin zur Aorta 9 beschreibt, gelangt ein gewisser Volumenstrom über den physiologischen Weg durch die Aortenklappe 19 in die Aorta 9. Das Herz-zeit-Volumen bzw. der Gesamtvolumenstrom ($Q_{HZV}$) vom Ventrikel 18 hin zur Aorta 9 ist demnach üblicherweise die Summe aus Pumpenvolumenstrom ($Q_p$) und Aortenklappen-Volumenstrom ($Q_a$).

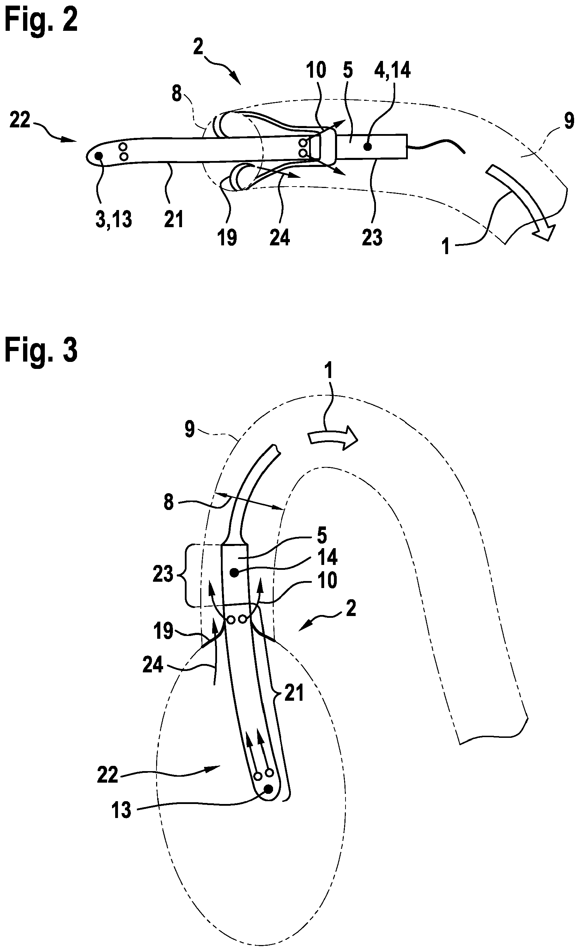

Fig. 2 zeigt schematisch ein implantiertes, vaskuläres Unterstützungssystem 2 in Aortenklappenposition. Zur näheren Veranschaulichung wird gleichzeitig auch auf die schematisch Anordnung des Unterstützungssystems 2 gemäß Fig. 3 Bezug genommen, wobei die Bezugszeichen einheitlich in allen Figuren verwendet werden.

Das Unterstützungssystem 2 ist hier beispielhaft ein linksventrikuläres Herzunterstützungssystem (LVAD).

Das Unterstützungssystem hat eine schlauchartige längliche Struktur mit einem Kanülenabschnitt, in dem als Kanüle eine Einlaufkanüle 21 ausgebildet ist, und weist einen an den Kanülenabschnitt angeschlossenen Motorgehäuseabschnitt, in dem sich ein Elektromotor 5 in einem Motorgehäuse 23 befindet.

Das Unterstützungssystem 2 ragt aus der Aorta 9 durch die Aortenklappen 19 distal in den Ventrikel 18. Das Unterstützungssystem 2 hat hier beispielhaft eine Einlaufkanüle 21, die in den Ventrikel 18 hineinragt. Durch die Einlaufkanüle 21 wird ein Fluid-Volumenstrom 10 unter Verwendung eines Elektromotors 5 des Unterstützungssystems 2, der in dem Unterstützungssystem 2 eine Strömungsmaschine in Form einer Pumpe antreibt, aus dem Ventrikel 18 in die Aorta 9 gefördert, beispielsweise gepumpt. Daher wird der Fluid-Volumenstrom 10 auch als Pumpenvolumenstrom ($Q_p$) bezeichnet, der nur den Fluss durch das Unterstützungssystem 2 selbst quantifiziert.

Zudem ist in Fig. 2 und Fig. 3 zu erkennen, dass ein gewisser Aortenklappen-Volumenstrom 24 über den physiologischen Weg durch die Aortenklappe 19 in die Aorta 9 gelangt. Das Herz-zeit-Volumen bzw. der im Bereich des Unterstützungssystems 2 durch eine Querschnittsgeometrie 8 der Aorta 9 hindurchtretende Fluid-Gesamtvolumenstrom 1 ($Q_{HZV}$) vom Ventrikel 18 hin zur Aorta 9 ist demnach die Summe aus Fluid-Volumenstrom 10 ($Q_p$) und Aortenklappen-Volumenstrom 24 ($Q_a$). Dies wird durch nachstehende Gleichung (1) beschrieben.

$$Q_{HZV} = Qp + Qa \qquad (1)$$

Das Unterstützungssystem 2 umfasst einen Referenztemperatursensor 13 zur Bestimmung einer Referenztemperatur 3 eines Fluids, hier beispielhaft Blut. Ferner umfasst das Unterstützungssystem 2 einen Elektromotor 5 sowie einen Motortemperatursensor 14 zur Bestimmung einer Motortemperatur 4 des Elektromotors 5. Zudem hat das Unterstützungssystem 2 einen Stromsensor (hier nicht dargestellt) zur Bestimmung der thermischen Verlustleistung (hier nicht dargestellt) des Elektromotors 5.

Der Motortemperatursensor 14 ist beispielhaft in einem Motorgehäuse 23 integriert, in dem die thermische Verlustleistung des Elektromotors 5 an das umgebende Fluid abgeführt wird. Der Motortemperatursensor 14 ist so eingerichtet und angeordnet, dass er die Motortemperatur 4 messen kann. Hierzu kann der Motortemperatursensor 14 so eingerichtet und angeordnet sein, dass er eine Oberflächentemperatur des Motorgehäuses 23 oder eine Temperatur des Stators (hier nicht dargestellt) des Elektromotors 5 misst. Hierbei kann die Temperatur des Stators durch eine Innentemperatur im Motorgehäuse 23 zwischen Motorgehäuse 23 und Wicklungspaket (hier nicht dargestellt) angenähert werden. Alternativ kann auch die Temperatur im Wicklungspaket direkt gemessen werden.

Der Referenztemperatursensor 13 erfasst die Referenztemperatur 3, die hier beispielhaft die Hintergrund-Bluttemperatur ist. Dazu ist der Referenztemperatursensor 13 im thermisch unbeeinflussten Blutfluss vor den die Wärmequelle darstellenden Elektromotor 5 platziert, hier beispielhaft im Bereich vor dem Elektromotor 5. Hierfür ist der Referenztemperatursensor 13, wie die Fig. 2 zeigt, in einem von dem Motorgehäuseabschnitt beabstandeten Bereich des Kanülenabschnitts an einem distalen Ende der Einlaufkanüle 21 angeordnet, d.h. dort, wo das Blut aus einem Ventrikel in die Einlaufkanüle 21 strömt.

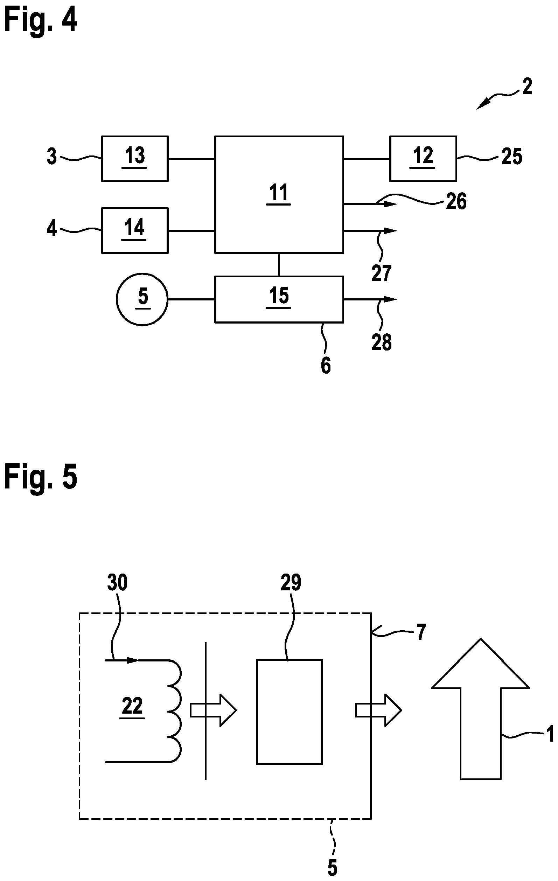

Fig. 4 zeigt schematisch eine Komponentenarchitektur eines Unterstützungssystems 2. Das Unterstützungssystem 2 umfasst einen Referenztemperatursensor 13 zur Bestimmung einer Referenztemperatur 3 eines Fluids, hier beispielhaft Blut. Ferner umfasst das Unterstützungssystem 2 einen Elektromotor 5 sowie einen Motortemperatursensor 14 zur Bestimmung einer Motortemperatur 4 des Elektromotors 5. Zudem hat das Unterstützungssystem 2 einen Stromsensor 15 zur Bestimmung der thermischen Verlustleistung 6 des Elektromotors 5. Hierzu ermittelt der Stromsensor 15 beispielhaft den Stromfluss (hier nicht dargestellt) durch den Motor 5 und rechnet diesen in die thermische Verlustleistung 6 um. Das Unterstützungssystem 2 umfasst gemäß der Darstellung nach Fig. 4 ferner eine Verarbeitungseinheit 11, die eingerichtet ist zur Bestimmung eines Fluid-Gesamtvolumenstroms (hier nicht dargestellt) im Bereich des Unterstützungssystems 2 unter Verwendung der Referenztemperatur 3, der Motortemperatur 4 und der thermischen Verlustleistung 6 des Elektromotors 5. Zudem hat das Unterstützungssystem 2 einen elektronisch lesbaren Speicher 12 mit Kalibrierdaten 25.

Die Messdaten des Referenztemperatursensors 13, des Motortemperatursensors 14 sowie des Stromsensors 15 werden an die Verarbeitungseinheit 11 übertragen. Die Verarbeitungseinheit 11 verarbeitet die Messdaten mit Kalibrierdaten 25 aus dem Speicher 12 zur Blutflussgeschwindigkeit bzw. dem (Gesamt-)Blutvolumen-Strom. Weiterhin weist die Verarbeitungseinheit 11 einen Ausgang 26 zu einer Kommunikationseinheit (hier nicht dargestellt), einen Ausgang 27 zu einer Stromversorgung (hier nicht dargestellt) und einen Ausgang 28 zu einer Motorsteuerung (hier nicht dargestellt) auf.

Fig. 5 zeigt schematisch eine Veranschaulichung eines beispielhaften Wärmeflusses (horizontale Pfeile) durch den Elektromotor 5 hin zu der Fluidströmung (vertikaler Pfeil) bzw. dem Fluid-Gesamtvolumenstrom 1. Der Elektromotor 5 weist hier beispielhaft einen beweglich gelagerten Rotor (hier nicht dargestellt) und ein durch einen Luftspalt außen abgesetztes stationäres Wicklungspaket 22 auf, das in Verbindung zum Stator 29 steht. Somit veranschaulicht Fig. 5 mit anderen Worten schematisch die Wärmeleitübergänge von dem Wicklungspaket 22 des Elektromotors 5 über den Stator 29 zum Blutfluss. Die Verlustmechanismen im Elektromotor 5 betreffen in erster Linie die Joul'schen Stromwärmeverluste Pv (siehe nachstehende Gleichung (2)).

$$P_V = R_{TW} \cdot I^2 \qquad (2)$$

Hierbei bezeichnet $R_{TW}$ den Wicklungswiderstand des Wicklungspakets 22 bei der Betriebstemperatur Tw. Der Wicklungswiderstand $R_{TW}$ ist im Fall von Kupfer linear von der Wicklungstemperatur Tw abhängig. Dies beschreibt die nachstehende Gleichung (3):

$$R_{TW} = R_{25} \cdot (1 + \alpha_{Cu}(T_W - 25)) \qquad (3)$$

mit dem Wicklungswiderstand $R_{25}$ bei 25°C, der Wicklungs-Betriebstemperatur $T_W$ und der Konstante $\alpha_{Cu} = 0{,}0039K^{-1}$.

Darüber hinaus treten noch Eisenverluste auf, beispielsweise Magnetisierungsverluste nach nachstehender Gleichung (4):

$$P_{V,magn} = \pi/30 \cdot M_{Magn} \cdot n \qquad (4)$$

und Wirbelstromverluste im Rückschlussmaterial des Stators nach nachstehender Gleichung (5):

$$P_{V,Wirbel} = const \cdot n^2 \qquad (5)$$

mit der Umdrehungszahl n des Motors und dem magnetischen Reibmoment $M_{Magn}$. Zusätzlich treten Lagerverluste aus der Lagerung des Motors auf, die in der Regel vernachlässigt werden können.

Der Wärmewiderstand zwischen einer Wärmequelle und einer Wärmesenke wird in Kelvin pro Watt gemessen (K/W). Der bestimmende Wärmeleitmechanismus zwischen Wicklungspaket und Blutfluss ist hier Wärmeleitung durch die Schichten des Motors nach außen, wie Fig. 5 zeigt. Zur Bestimmung der Temperaturen sind die Wärmekapazitäten der einzelnen vom Wärmefluss durchquerten Bauteile, sowie die jeweiligen Wärmeübergangswiderstände erforderlich. Da hinreichend angenommen werden kann, dass sich der Elektromotor in stationärem Betrieb und damit in thermischem Gleichgewicht befindet, können die Wärmekapazitäten vernachlässigt werden. Alle notwendigen Parameter sind vorab bestimmbar und können in einer Verarbeitungseinheit hinterlegt werden.

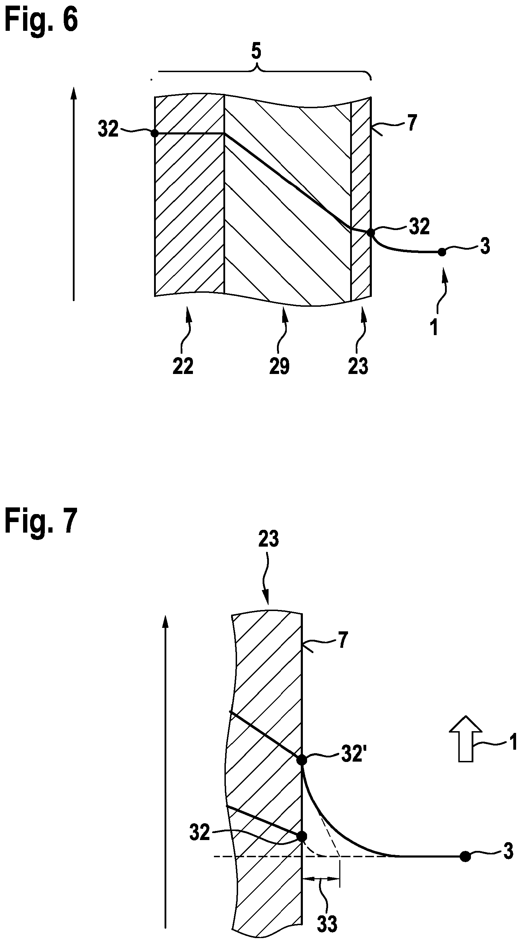

Fig. 6 zeigt schematisch eine Veranschaulichung eines Temperaturverlaufs entlang der Materialschichtenfolge vom Wicklungspaket 22 über den Stator 29 und das Motorgehäuse 23 zum Fluid-Gesamtvolumenstrom 1. Hierbei stellt Fig. 6 eine sich im thermischen Gleichgewicht ergebende Temperaturverteilung für einen Wärmefluss nach Fig. 5 dar. Die höchste Temperatur liegt in der Wärmequelle, dem vom elektrischen Strom durchflossenen Wicklungspaket 22 vor. Die Wicklungstemperatur 31 (Formelzeichen Tw) des Wicklungspakets 22 ist in Fig. 6 daher die höchst Temperatur. Zur Vereinfachung wurde hier eine konstante Wärmeverteilung über die gesamte Dicke des Wicklungspaketes 22 angenommen. Aufgrund der endlichen thermischen Leitfähigkeit von Stator- und Gehäusematerial ergibt sich ein lineares Temperaturgefälle über den Stator 29 und das Motorgehäuse 23, bzw. für den nicht-vereinfachten Fall eines zylinderförmigen Motorgehäuses 23 ein logarithmisches Temperaturgefälle.

Die sich im Wicklungspaket 23 einstellende Wicklungstemperatur 31 (Formelzeichen Tw) beträgt in der vereinfachten Prinzipbetrachtung:

$$T_W = T_A + (R_{th1} + R_{th2}) \cdot P_v \qquad (6)$$

$$T_W = T_A + (R_{th1} + R_{th2}) \cdot R_{TW} \cdot I^2 \qquad (7)$$

$$T_W = T_A + (R_{th1} + R_{th2}) \cdot R_{25} \cdot (1 + \alpha_{Cu}(T_W - 25°C)) \qquad (8)$$

$$T_W = T_A + \frac{(R_{th1} + R_{th2}) \cdot R_{25} \cdot I^2}{1 - \alpha_{Cu} \cdot (R_{th1} + R_{th2}) \cdot R_{25} \cdot I^2} \qquad (9)$$

Hierbei sind der elektrische Stromfluss 30 (Formelzeichen I) und die Oberflächentemperatur 32 (Formelzeichen $T_A$) die einzigen variablen Parameter. $R_{th1}$ beschreibt den Wärmewiderstand zwischen dem Wicklungspaket 22 und dem Stator 29. $R_{th2}$ beschreibt den Wärmewiderstand zwischen dem Stator 29 und der Fluidströmung. Der Stromfluss 30 (Formelzeichen I) kann durch Messung mit dem Stromsensor 15, bspw. in einem Steuergerät des Stromsensors ermittelt werden und ist somit genau bekannt. Die Oberflächentemperatur 32 (Formelzeichen $T_A$) bezeichnet die Temperatur auf einer Oberfläche 7 des Elektromotors 5, an der das Fluid entlangströmt. Mit anderen Worten ist die Oberfläche 7 in der Blutströmung.

Fig. 7 zeigt schematisch eine weitere Veranschaulichung eines Temperaturverlaufs. Fig. 7 stellt hierbei eine Detailansicht der Darstellung nach Fig. 6 im Bereich der Oberfläche 7 bei zwei unterschiedlichen Strömungsgeschwindigkeiten dar. Mit anderen Worten ausgedrückt veranschaulicht Fig. 7 die Abhängigkeit der Temperatur(en) (Oberflächentemperatur und damit auch Stator- und damit auch Wicklungspakettemperatur) von der Strömungsgeschwindigkeit des Fluidstromes bzw. des Blutes.

Wie in Fig. 7 gezeigt, stellt sich nahe der Oberfläche 7 ein Flüssigkeitsfilm der Dicke 33 ein. Die Dicke 33 des Flüssigkeitsfilms und der Temperaturunterschied $T_A$ - $T_B$ zwischen der Oberflächentemperatur 32 (Formelzeichen $T_A$) und der Referenztemperatur 3 (Formelzeichen $T_B$), die die Hintergrundtemperatur des Fluids (des Bluts) darstellt, ist von der Strömungsgeschwindigkeit des Fluids abhängig, wie dies in Fig. 7 veranschaulicht ist. Gemäß der Darstellung nach Fig. 7 führt eine geringere Strömungsgeschwindigkeit des Fluids entlang der Oberfläche 7 zu einer höheren Oberflächentemperatur 32' als der Oberflächentemperatur 32, die sich bei einer vergleichsweise höheren Strömungsgeschwindigkeit einstellt.

Der Wärmestrom durch den Flüssigkeitsfilm beträgt

$$\dot{Q} = \alpha_B(T_B - T_A)A \qquad (10)$$

mit dem Wärmeübergangskoeffizienten $\alpha_B$ von der Gehäuseoberseite zum Blut und der benetzten Fläche A der Oberfläche 7. Der Wärmeübergangskoeffizient ist definiert als

$$\alpha_B = \frac{Nu\lambda}{L} \qquad (11)$$

mit der dimensionslosen Nusseltzahl $Nu$, der Wärmeleitfähigkeit $\lambda$ des Fluids (hier: Blut) und einer Referenzlänge L, bei der es sich beispielsweise um einen Rohrdurchmesser handeln kann. Für die über die Körperoberfläche gemittelte Nusseltzahl gilt weiterhin, dass sie eine Funktion der dimensionslosen Reynoldszahl $Re$ und Prandlzahl $Pr$ ist:

$$Nu = f(Re, Pr) \qquad (12)$$

Diese können jeweils in Abhängigkeit von der Geometrie und der Strömung (Re und Pr) bzw. in Abhängigkeit der Fluideigenschaften (Pr) berechnet und im Kalibrierdatenspeicher abgelegt werden. Die Reynoldszahl ist definiert als

$$Re = \frac{uL}{v} \qquad (13)$$

mit der charakteristischen Länge L (z.B. Rohrdurchmesser), der kinematischen Viskosität des Fluids v und der gesuchten Strömungsgeschwindigkeit u. Die Prandlzahl ist eine reine Stoffgröße und gegeben durch

$$Pr = \frac{v}{a} \qquad (14)$$

mit der Temperaturleitfähigkeit a des Fluids. Werden die Definitionen in den konvektiven Wärmestrom durch den Flüssigkeitsfilm (Gleichung (10) eingesetzt, erhält man den Zusammenhang zwischen bekanntem Wärmestrom $\dot{Q}$ und der gesuchten Flussgeschwindigkeit u. Das Ergebnis dieses Einsetzens ist in Gleichung (15) unten gezeigt. Der Wärmestrom $\dot{Q}$ ist hierbei aus einer Energiebilanz bekannt. Aus der Energiebilanz für den hier betrachteten stationären Fall folgt, dass der Wärmestrom $\dot{Q}$ (betragsmäßig) im Wesentlichen der thermischen Verlustleistung 6 (Formelzeichen Pv) entspricht.

Die Oberflächentemperatur 32 (Formelzeichen $T_A$) kann hier beispielsweise mittels des Motortemperatursensors 14 direkt an der Oberfläche 7 gemessen werden oder der Motortemperatursensor 14 kann eine Temperatur im Motorinneren messen und die Oberflächentemperatur 32 (Formelzeichen $T_A$) wird aus dem logarithmischen Temperaturzusammenhang zur Temperaturverteilung im Motorgehäuse (vgl. Fig. 6 und 7) ermittelt. Die Referenztemperatur 3 (Formelzeichen $T_B$) wird durch den Referenztemperatursensor 13 bestimmt. Die Parameter L, v, a, $\lambda$ und A sind in der Regel als Kalibrierdaten im System hinterlegt.

$$\dot{Q} = \frac{f(\frac{uL}{v}, \frac{v}{a})\lambda}{L}(T_A - T_B)A \qquad (15)$$

Bei bekannter Querschnittsgeometrie 8 der Aorta 9 des Patienten im Bereich des Unterstützungssystems (ermittelbar z.B. durch Ultraschall, Computertomografie oder Magnetresonanztomografie) kann aus der so bestimmten Strömungsgeschwindigkeit u der Fluid-Gesamtvolumenstrom 1 (Formelzeichen $Q_{HZV}$) bestimmt werden. Der entsprechende Zusammenhang ist in nachfolgender Gleichung (16) angegeben:

$$Q_{HZV} = k(u)uO \qquad (16)$$

Hierbei ist k(u) ein vom Strömungsprofil abhängiger Kalibrierfaktor, u die berechnete Strömungsgeschwindigkeit und O der gemessene Aortenquerschnitt (vgl. Querschnittsgeometrie 8).

Die hier vorgeschlagene Lösung ermöglicht insbesondere einen der nachstehenden Vorteile:

- Vollimplantierte, insbesondere pumpenintegrierte und/oder automatische Bestimmung von $Q_{HZV}$ statt nur $Q_p$.
- Durch anemometrisches Messverfahren mit Nutzung der Abwärme eines VAD-Motors statt Verwendung eines zusätzlichen Heizelements findet kein zusätzlicher Wärmeeintrag in den Organismus statt.
- Dadurch wird zudem eine zusätzliche Stromaufnahme vermieden, wodurch die Batterielaufzeit autonomer Systeme verlängert wird.

**Patentansprüche**

1. Verfahren zur Bestimmung eines Fluid-Gesamtvolumenstroms (1) im Bereich eines implantierten, vaskulären Unterstützungssystems (2), umfassend folgende Schritte:

   a) Bestimmen einer Referenztemperatur (3) des Fluids,
   b) Bestimmen einer Motortemperatur (4) eines Elektromotors (5) des Unterstützungssystems (2),

c) Bestimmen der thermischen Verlustleistung (6) des Elektromotors (5),

d) Ermitteln des Fluid-Gesamtvolumenstroms (1) unter Verwendung der Referenztemperatur (3), der Motortemperatur (4) und der thermischen Verlustleistung (6) des Elektromotors (5).

**2.** Verfahren nach Anspruch 1, **dadurch gekennzeichnet, dass** die Referenztemperatur (3) vor einer Erwärmung des Fluids durch den Elektromotor (5) gemessen wird.

**3.** Verfahren nach Anspruch 1 oder 2, **dadurch gekennzeichnet, dass** die Motortemperatur (4) des Elektromotors (5) an einer Oberfläche (7) gemessen wird, an der das Fluid entlangströmt oder die Motortemperatur (4) des Elektromotors (5) im Motorinneren gemessen wird.

**4.** Verfahren nach einem der vorhergehenden Ansprüche, **dadurch gekennzeichnet, dass** in Schritt d) in Abhängigkeit von Kalibrierdaten, der Referenztemperatur (3), der Motortemperatur (4) und der thermischen Verlustleistung (6) des Elektromotors (5) eine Strömungsgeschwindigkeit des Fluids bestimmt wird.

**5.** Verfahren nach einem der vorhergehenden Ansprüche, **dadurch gekennzeichnet, dass** in Schritt d) weiterhin eine ermittelte Querschnittsgeometrie (8) einer Aorta (9) im Bereich des implantierten, vaskulären Unterstützungssystems (2) berücksichtigt wird.

**6.** Verfahren nach einem der vorhergehenden Ansprüche, **dadurch gekennzeichnet, dass** weiterhin ein Fluid-Volumenstrom (10) bestimmt wird, der durch das Unterstützungssystem (2) strömt.

**7.** Verarbeitungseinheit (11), eingerichtet zur Durchführung des Verfahren nach einem der Vorhergehenden Ansprüche und aufweisend einen Speicher (12), in dem Kalibrierdaten hinterlegt sind.

**8.** Implantierbares, vaskuläres Unterstützungssystem (2), umfassend:

- einen Elektromotor (5),
- einen Motortemperatursensor (14) zur Bestimmung einer Motortemperatur (4) des Elektromotors (5),
- einen Stromsensor (15) zur Bestimmung zumindest des Stromflusses durch den Elektromotor oder der thermischen Verlustleistung (6) des Elektromotors (5);

**gekennzeichnet durch:**

einen Referenztemperatursensor (13) zur Bestimmung einer Referenztemperatur (3) eines Fluids.

**9.** Unterstützungssystem nach Anspruch 8, **gekennzeichnet durch** eine Verarbeitungseinheit (11), eingerichtet zur Bestimmung eines Fluid-Gesamtvolumenstroms (1) im Bereich des Unterstützungssystems (2) unter Verwendung der Referenztemperatur (3), der Motortemperatur (4) und der thermischen Verlustleistung (6) des Elektromotors (5) und/oder wobei der Motortemperatursensor (14) eine Temperatur eines Stators des Elektromotors (5) misst.

**10.** Unterstützungssystem nach Anspruch 8 oder 9, **gekennzeichnet durch** eine mittels des Elektromotors (5) angetriebene Strömungsmaschine für das Fördern des Fluids und eine Kanüle (21), durch die das Fluid zu der Strömungsmaschine geführt werden kann.

**11.** Unterstützungssystem nach Anspruch 10, **dadurch gekennzeichnet, dass** die Kanüle (21) für das Führen von Fluid in Form von Blut aus einem Ventrikel (18) eines Herzens in eine Aorta (9) ausgelegt ist und/oder dass der Referenztemperatursensor (13) an oder in der Nähe eines von der Strömungsmaschine beabstandeten Bereichs der Kanüle (21) angeordnet ist und/oder dass der Referenztemperatursensor (13) an oder in der Nähe eines dem Elektromotor (5) abgewandten Bereichs der Kanüle (21) angeordnet ist.

**12.** Unterstützungssystem nach einem der Ansprüche 10 oder 11, **gekennzeichnet durch** eine schlauchartige längliche Struktur mit einem Kanülenabschnitt, in dem die Kanüle (21) ausgebildet ist, und mit einem an den Kanülenabschnitt angeschlossenen Motorgehäuseabschnitt, in dem der Elektromotor (5) in einem Motorgehäuse (23) angeordnet ist.

**13.** Unterstützungssystem nach Anspruch 12, **dadurch gekennzeichnet, dass** der Referenztemperatursensor (13) in einem von dem Motorgehäuseabschnitt beabstandeten Bereich des Kanülenabschnitts angeordnet ist und/oder dass das Motorgehäuse (23) in der Aorta (9) mit Blut umströmbar ist.

**14.** Unterstützungssystem nach einem der Ansprüche 8 bis 11, **dadurch gekennzeichnet, dass** der Elektromotor (23) in einem in der Aorta (9) mit Blut umströmbaren Motorgehäuse (23) angeordnet ist.

**15.** Unterstützungssystem nach einem der Ansprüche 12 bis 14, **dadurch gekennzeichnet, dass** der Motortemperatursensor (14) eine Oberflächentemperatur des Motorgehäuses (23) misst.

**Claims**

**1.** Method for determining a total fluid volume flow (1) in the region of an implanted vascular support system (2), comprising the following steps:

a) determining a reference temperature (3) of the fluid,
b) determining a motor temperature (4) of an electric motor (5) of the support system (2),
c) determining the thermal power dissipation (6) of the electric motor (5),
d) ascertaining the total fluid volume flow (1) by using the reference temperature (3), the motor temperature (4) and the thermal power dissipation (6) of the electric motor (5).

**2.** Method according to claim 1, **characterized in that** the reference temperature (3) is measured before the fluid is heated by the electric motor (5).

**3.** Method according to claim 1 or claim 2, **characterized in that** the motor temperature (4) of the electric motor (5) is measured on a surface (7) along which the fluid flows or the motor temperature (4) of the electric motor (5) is measured inside the motor.

**4.** Method according to any of the preceding claims, **characterized in that,** in step d), a flow rate of the fluid is determined on the basis of calibration data, the reference temperature (3), the motor temperature (4) and the thermal power dissipation (6) of the electric motor (5).

**5.** Method according to any of the preceding claims, **characterized in that,** in step d), an ascertained cross-sectional geometry (8) of an aorta (9) in the region of the implanted vascular support system (2) is also taken into account.

**6.** Method according to any of the preceding claims, **characterized in that** a fluid volume flow (10) which flows through the support system (2) is also determined.

**7.** Processing unit (11) configured to carry out the method according to any of the preceding claims and comprising a memory (12) in which calibration data are stored.

**8.** Implantable vascular support system (2), comprising:

- an electric motor (5),
- a motor temperature sensor (14) for determining a motor temperature (4) of the electric motor (5),
- a current sensor (15) for determining at least the current flow through the electric motor or the thermal power dissipation (6) of the electric motor (5);

**characterized by:**
a reference temperature sensor (13) for determining a reference temperature (3) of a fluid.

**9.** Support system according to claim 8, **characterized by** a processing unit (11) configured to determine a total fluid volume flow (1) in the region of the support system (2) by using the reference temperature (3), the motor temperature (4) and the thermal power dissipation (6) of the electric motor (5) and/or wherein the motor temperature sensor (14) measures a temperature of a stator of the electric motor (5).

**10.** Support system according to claim 8 or claim 9, **characterized by** a flow machine, driven by the electric motor (5), for conveying the fluid, and a cannula (21) through which the fluid can be conducted to the flow machine.

**11.** Support system according to claim 10, **characterized in that** the cannula (21) is designed for conducting fluid in the form of blood from a ventricle (18) of a heart into an aorta (9) and/or **in that** the reference temperature sensor (13) is arranged on or near a region of the cannula (21) spaced apart from the flow machine and/or **in that** the reference temperature sensor (13) is arranged on or near a region of the cannula (21) remote from the electric motor (5).

12. Support system according to either of claims 10 or 11, **characterized by** a tube-like elongate structure having a cannula portion, in which the cannula (21) is formed, and having a motor housing portion connected to the cannula portion, in which motor housing portion the electric motor (5) is arranged in a motor housing (23).

13. Support system according to claim 12, **characterized in that** the reference temperature sensor (13) is arranged in a region of the cannula portion spaced apart from the motor housing portion and/or **in that** blood can flow around the motor housing (23) in the aorta (9).

14. Support system according to any of claims 8 to 11, **characterized in that** the electric motor (23) is arranged in a motor housing (23) around which blood can flow in the aorta (9).

15. Support system according to any of claims 12 to 14, **characterized in that** the motor temperature sensor (14) measures a surface temperature of the motor housing (23).

**Revendications**

1. Procédé permettant de déterminer un débit volumique total de fluide (1) dans la zone d'un système de support (2) vasculaire implanté, comprenant les étapes suivantes:

   a) détermination d'une température de référence (3) du fluide,
   b) détermination d'une température de moteur (4) d'un moteur électrique (5) du système d'assistance (2),
   c) détermination de la puissance thermique dissipée (6) du moteur électrique (5),
   d) définition du débit volumique total de fluide (1) à l'aide de la température de référence (3), de la température de moteur (4) et de la puissance thermique dissipée (6) du moteur électrique (5).

2. Procédé selon la revendication 1, **caractérisé en ce que** la température de référence (3) est mesurée avant un chauffage du fluide par le moteur électrique (5).

3. Procédé selon la revendication 1 ou 2, **caractérisé en ce que** la température de moteur (4) du moteur électrique (5) est mesurée sur une surface (7) le long de laquelle le fluide s'écoule ou la température de moteur (4) du moteur électrique (5) est mesurée à l'intérieur du moteur.

4. Procédé selon l'une des revendications précédentes,
   **caractérisé en ce que,** à l'étape d), une vitesse d'écoulement du fluide est déterminée en fonction de données d'étalonnage, de la température de référence (3), de la température de moteur (4) et de la puissance thermique dissipée (6) du moteur électrique (5).

5. Procédé selon l'une des revendications précédentes,
   **caractérisé en ce que,** à l'étape d), une géométrie de section transversale (8) définie d'une aorte (9) dans la zone du système d'assistance (2) vasculaire implanté est en outre prise en compte.

6. Procédé selon l'une des revendications précédentes,
   **caractérisé en ce qu'**un débit volumique de fluide (10) est en outre déterminé, lequel s'écoule à travers le système d'assistance (2).

7. Unité de traitement (11), configurée pour mettre en oeuvre le procédé selon l'une des revendications précédentes et présentant une mémoire (12) dans laquelle sont stockées des données d'étalonnage.

8. Système de support (2) vasculaire implantable, comprenant :

   - un moteur électrique (5),
   - un capteur de température de moteur (14) permettant de déterminer une température de moteur (4) du moteur électrique (5),

- un capteur de courant (15) permettant de déterminer au moins le flux de courant à travers le moteur électrique ou la puissance thermique dissipée (6) du moteur électrique (5) ;

**caractérisé par** :
un capteur de température de référence (13) permettant de déterminer une température de référence (3) d'un fluide.

9. Système de support selon la revendication 8, **caractérisé par** une unité de traitement (11), configurée pour déterminer un débit volumique total de fluide (1) dans la zone du système d'assistance (2) à l'aide de la température de référence (3), de la température de moteur (4) et de la puissance thermique dissipée (6) du moteur électrique (5), et/ou dans lequel le capteur de température de moteur (14) mesure une température d'un stator du moteur électrique (5).

10. Système de support selon la revendication 8 ou 9, **caractérisé par** une turbomachine entraînée au moyen du moteur électrique (5) pour l'acheminement du fluide et une canule (21) à travers laquelle le fluide peut être guidé vers la turbomachine.

11. Système de support selon la revendication 10, **caractérisé en ce que** la canule (21) est conçue pour le guidage d'un fluide sous forme de sang hors d'un ventricule (18) d'un coeur dans une aorte (9) **et/ou en ce que** le capteur de température de référence (13) est disposé au niveau ou à proximité d'une zone de la canule (21) espacée de la turbomachine **et/ou en ce que** le capteur de température de référence (13) est disposé au niveau ou à proximité d'une zone de la canule (21) opposée au moteur électrique (5).

12. Système de support selon l'une des revendications 10 ou 11, **caractérisé par** une structure allongée en forme de tuyau comportant une section de canule dans laquelle la canule (21) est formée, et comportant une section de boîtier de moteur raccordée à la section de canule, dans laquelle section de boîtier de moteur le moteur électrique (5) est disposé dans un boîtier de moteur (23).

13. Système de support selon la revendication 12, **caractérisé en ce que** le capteur de température de référence (13) est disposé dans une zone de la section de canule espacée de la section de boîtier de moteur **et/ou en ce que** le boîtier de moteur (23) peut être entouré de sang dans l'aorte (9).

14. Système de support selon l'une des revendications 8 à 11,
**caractérisé en ce que** le moteur électrique (23) est disposé dans un boîtier de moteur (23) pouvant être entouré de sang dans l'aorte (9).

15. Système de support selon l'une des revendications 12 à 14, **caractérisé en ce que** le capteur de température de moteur (14) mesure une température de surface du boîtier de moteur (23).

# Fig. 1a

# Fig. 1b

# Fig. 2

# Fig. 3

# Fig. 4

# Fig. 5

# Fig. 6

5

32

7

32

3

1

22    29    23

# Fig. 7

23

7

32'

1

32

3

33

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012112378 A2
- WO 9843688 A1